# EUROPEAN PATENT APPLICATION

(11) **EP 4 130 227 A1**
(43) Date of publication of application: **08.02.2023**
(21) Application number: 21774130.5
(22) Date of filing: 24.03.2021
(51) Int. Cl.: C12M 1/00, B65D 77/04, B65D 81/26, C12M 1/12

(54) **IRRADIATED RESIN MOLDING**

(30) Priority: 25.03.2020 JP 2020054462
(71) Applicant: Nissan Chemical Corporation, Tokyo 103-6119 (JP)
(72) Inventor: HIROI Yoshiomi, Funabashi-shi, Chiba 274-0052 (JP); SUZUKI Kohei, Funabashi-shi, Chiba 274-0052 (JP); HIROI Miya, Funabashi-shi, Chiba 274-0052 (JP)
(74) Representative: dompatent von Kreisler Selting Werner - Partnerschaft von Patent- und Rechtsanwälten mbB
(86) International application number: PCT/JP2021/012429
(87) International publication number: WO 2021/193780

(57) **Abstract**

A resin molding and a method of producing the same in which sterilizability thereof is secured and discoloration (yellowing) thereof is reduced even when the resin molding is radiation-irradiated under anaerobic conditions (deoxidation conditions), are provided.

The radiation-irradiated and packaged resin molding, comprising that said resin molding is primarily packaged with a packaging material having oxygen permeability and then secondarily packaged with an oxygen impermeable packaging material together with a deoxidizer.

Further the method of producing a radiation-irradiated and packaged resin molding, comprising a step of primarily packaging a resin molding before radiation irradiation with a packaging material having oxygen permeability, a step of subsequently secondarily packaging with an oxygen impermeable packaging material together with a deoxidizer, and then conducting radiation irradiation. It is preferable to comprise a step of opening the secondary packaging under the atmosphere, following said radiation irradiation step.

## Description

### Technical Field

The present invention relates to a radiation-irradiated resin molding, preferably the resin molding radiation-irradiated under anaerobic conditions, such as sterilized cell culture vessel.

### Background Technology

The resin moldings (moldings made of plastic) are widely used as materials for various substrates, because of advantages such as manufacturing easiness, low cost, and light weight. For example, in order to use the resin molding as cell culture vessels, it is necessary to sterilize them prior to the use. As a method of sterilization, radiation sterilization by gamma rays (γ rays), electron beams, etc., is used, while yellow denaturation (yellowing) derived from antioxidant contained in the molding may occur in transparent resin molding. In particular, the radiation sterilization of resin compositions is often conducted under anaerobic conditions (deoxidation conditions) in order to prevent deterioration of the resin molding caused by oxygen free radicals generated by irradiation, but it is known that the problem of yellowing becomes conspicuous when radiation sterilization is conducted under anaerobic conditions (deoxidation conditions).

Patent Document 1 describes the method of sterilization, in which a polycarbonate resin molding is subjected to ionizing radiation sterilization, then subjected to heat treatment at a temperature of 30 ~ 130°C under deoxidation atmosphere, with yellowing being extremely small and with deterioration in physical properties being also small.

### Prior art documents

### Patent Documents

Patent Document 1. Japanese Unexamined Patent Application Publication No. 2001 -294690

### Disclosure of the Invention

### Problems to be solved by the Invention

The present invention provides the radiation-irradiated resin molding, preferably a sterilized resin molding and a method of producing the same, in which the sterilizability thereof is ensured and the discoloration thereof is reduced, even when the radiation irradiation thereto has been conducted under anaerobic conditions (deoxidation conditions).

### Means for Solving the Problem

The present invention comprises the following:
(1) A radiation-irradiated and packaged resin molding, comprising that said resin molding is primarily packaged with a packaging material having oxygen permeability, and then secondarily packaged with an oxygen impermeable packaging material together with a deoxidizer.
(2) The resin molding according to item (1), wherein the inside of the primary packaging is radiation-irradiated under anaerobic conditions.
(3) The resin molding according to item (1) or (2), wherein the inside of the primary packaging is radiation-irradiated under residual oxygen concentration of less than 0.5%.
(4) The resin molding according to any one of items (1) to (3), wherein said secondary packaging is opened under the atmosphere after the radiation irradiation.
(5) The resin molding according to item (4), wherein discoloration after said radiation irradiation is reduced.
(6) The resin molding according to any one of items (1) to (5), wherein the packaging material of said primary packaging comprises nonwoven fabric made of high density polyethylene.
(7) The resin molding according to any one of items (1) to (6), wherein the packaging material of said primary packaging comprises a combination of the packaging material having oxygen permeability and the oxygen impermeable packaging material.
(8) The resin molding according to any one of items (1) to (7), wherein the packaging material of said primary packaging comprises a combination of nonwoven fabric made of high density polyethylene having oxygen permeability and the oxygen impermeable packaging material.
(9) The resin molding according to any one of items (1) to (8), wherein said oxygen impermeable packaging material is selected from the group consisting of nylon, polyvinyl alcohol, low density polyethylene, polypropylene, polystyrene, polyvinyl chloride, polyacrylic acid, polyacrylic ester, polyacrylonitrile, polyvinyl acetate, polyacrylamide, polydimethylsiloxane and polyethylene terephthalate.
(10) The resin molding according to any one of items (1) to (9), wherein said radiation is selected from γ-rays, X-rays or electron rays.
(11) A method of producing a radiation-irradiated and packaged resin molding, comprising a step of primarily packaging a resin molding with a packaging material having oxygen permeability, and a step of subsequently secondarily packaging them with an oxygen impermeable packaging material together with a deoxidizer, thereafter conducting radiation irradiation.
(12) The producing method according to item (11), wherein the step of the radiation irradiation is conducted under anaerobic conditions.
(13) The producing method according to item (11) or (12), comprising a step of opening the secondary package under the atmosphere, following said step of the radiation irradiation.
(14) The producing method according to item (13), wherein discoloration after the radiation irradiation is reduced.
(15) The resin composition according to any one of items (1) to (10), wherein said resin molding is a cell culture vessel.
(16) The method of producing according to any one of items (11) to (14), wherein said resin molding is a cell culture vessel.
(17) The resin composition according to any one of items (1) to (10) and (15), wherein a bioburden of said resin molding is 1 cfu/g or less.
(18) The method of producing according to any one of items (11) to (14) and (16), wherein a bioburden of said resin molding after the step of the radiation irradiation is 1 cfu/g or less.
(19) A method of sterilizing a resin molding, comprising:
   a step of primarily packaging a resin molding with a packaging material having oxygen permeability;
   a step of secondarily packaging them with an oxygen impermeable packaging material together with a deoxidizer; and
   a step of conducting radiation irradiation.
(20) The method according to item (19), wherein the radiation irradiation is conducted with the inside of the primary packaging being under anaerobic conditions.
(21) The method according to item (19) or (20), comprising a step of opening the secondary package under the atmosphere, following the step of the radiation irradiation.
(22) The method according to item (21), wherein the discoloration of the resin composition after radiation irradiation is reduced.

### Effects of the Invention

As the primary packaging of the resin molding which is subject to be radiation-irradiated, preferably the resin molding for treatment of sterilization to be conducted, the oxygen permeable packaging material is used. When the primary packaging is subjected to the secondary packaging with an oxygen impermeable packaging material together with the deoxidizer and then radiation-irradiated at a target dose, the transparent resin molding usually turns yellow. Thereafter, by opening only the secondary packaging and storing them for any period of time (e.g., 1 hour or more) at ambient temperature and under the atmosphere, the resin molding (e.g., cell culture vessel) in their original color without discoloration can be obtained while maintaining the sterility of the transparent resin molding.

### Brief Description of Drawings

FIG. 1 is a picture showing the primary packaging described in Example 1. The white tape vertically attached to the packaging material in the photograph is a 20 mm width tape of Tyvek ^{®} (product number: 1073 B) (manufactured by DuPont) having oxygen permeability.
FIG. 2 is a schematic drawing showing the secondary packaging of the present application.
FIG. 3 is a picture of inverted type microscope of the plate obtained in Example 3 (the one in which the secondary package was opened under the atmosphere after γ-ray irradiation, and then the primary packaging was opened at least 24 hours after), showing a state of adhesion of cells in cell adhesion test.
FIG. 4 is a picture of inverted type microscope of the plate obtained in Example 7 (the one in which the primary packaging was opened at least 24 hours after γ-ray irradiation) showing a state of adhesion of cells in the cell adhesion test.

### Mode for Carrying Out the Invention

### <Resin molding>

The resin molding of the present application comprises that the resin molding is primarily packaged with the packaging material having oxygen permeability and is secondarily packaged with an oxygen impermeable packaging material together with the deoxidizer. The resin molding may become the sterilized moldings by the radiation irradiation at an intensity that allows for sterilization treatment. In the case of transparent resin molding, yellowing may occur due to the radiation irradiation under anaerobic conditions.

The term "oxygen permeability/oxygen impermeability" refers to as, for example, oxygen permeability such as degree that the inside of the primary packaging becomes sufficiently anaerobic conditions (deoxidation) by leaving them to stand at room temperature under the atmosphere for a sufficient period of time (e.g., 1 hour or more, 2 hours or more, 3 hours or more, 6 hours or more, 12 hours or more, 24 hours or more) after the secondary packaging as described above. Being secondarily packaged with the oxygen impermeable packaging material together with the deoxidizer makes the inside of primary packaging sufficiently anaerobic. After this condition is reached, for example radiation irradiation is conducted to sterilize.

As a specific example of the anaerobic condition above described, the oxygen concentration measured by the residual oxygen content meter (specific example is described in the Example) is below the detection sensitivity (e.g., less than 0.5%, less than 0.1%) or the oxygen concentration in which the oxygen sensing agent (specific example is described in the Example) shows deoxidation condition (e.g., less than 0.5%).

The term "transparent" of the present application refers to as being a state in which something on the opposite side and inside of an object can be seen through by visible light. It may be translucent such as cloudy or distorted, but can be seen through. Further, even if the resin molding of the present application contains fine particles and the like and scatters visible light, it may be sufficient that the resin molding transmits visible light.

In the resin molding of the present application, when the resin molding is normally colorless and transparent, yellowing is observed after undergoing the radiation irradiation step under anaerobic conditions. The resin molding of the present invention may also be the one which is colored after undergoing the radiation irradiation step under anaerobic conditions to such an extent that the reduction of discoloration can be recognized by the method for determining the reduction of discoloration described below.

Although the degree of color of the resin molding of the present invention is not particularly limited, it is preferable that the resin molding is colorless and transparent. The coloring method of the resin may be the one which is by a method known per se, such as mixing a dye thereinto.

Though the state in which a microorganism is completely killed and removed refers to as "an aseptic (state)", since it is difficult to guarantee complete aseptic in practice, "sterilization" is usually used to refer to the act of bringing the possibility of the growth of a microorganism as close to zero as possible. In sterilization of medical devices, "Sterility assurance level (Sterility Assurance level, SAL)" is used as an index indicating the probability of the presence of microorganisms, and the like on the medical device after sterilization, and sterility shall be assured by achieving 10 ⁻⁶ or less.

The term "sterilized" of the present application refers to as being sterilized to a level practically acceptable for use as, for example, cell culture vessel. For example, it refers to as being level in which the sterility assurance level (SAL) is at a level similar to 10 ⁻⁶ or less. Specifically, it means that the bioburden (bacterial count) contained in the resin molding or attached to the surface of the resin molding after sterilization by radiation irradiation is 1 cfu/g or less (cfu: colony forming units: the number of colonies that emerge when cultured).

The bioburden can be measured, for example, by the method of ISO 11737 -1 or JIS T 11737 -1 (the latest method thereof if the method was revised).

If the resin molding turns yellow after the radiation irradiation described above is conducted, by opening the secondary packaging under the atmosphere for, e.g., 1 hour or more, 2 hours or more, 3 hours or more, 6 hours or more, 12 hours or more, 24 hours or more, the discoloration (typically yellowing) of the resin molding is eliminated and the original transparency (typically clear and colorless) before the radiation irradiation is restored. That is, a resin composition in which discoloration (typically yellowing) after radiation irradiation described above is reduced can be obtained. Since the primary packaging usually remains to be maintained, when the intensity of radiation irradiation is strong enough to sterilize, and the primary packaging has porous materials (e.g., nonwoven fabric) having a physicochemical strength such degree that oxygen permeates without direct contamination of bacteria and microorganisms, the resin molding within the primary packaging shall be secured (retained) in a sterile state. This sterilization condition is maintained for at least 1 year as long as the primary packaging is not opened or the seal is well maintained.

The term "discoloration was reduced" means that the yellowness and yellowing degree of the resin molding before the radiation irradiation step and the resin molding with the secondary packaging opened after the radiation irradiation step are measured by a method in accordance with JIS K 7105, and ΔYI (degree of yellowing change) < 1. Alternatively, it refers to as the case in which 60% or more (3 persons or more in the case of 5 or more persons) of 5 or more healthy persons with no visual abnormality can estimate by visually observing that there is no discoloration of the resin molding before the radiation irradiation step described above and the resin molding with the secondary packaging opened after the radiation irradiation step.

As the resin molding of the present application, there may be a molding using a known resin. As the resin, there may be any of a natural resin or a derivative thereof, or a synthetic resin, and as the natural resin or the derivative thereof, there may be preferably used, cellulose, cellulose triacetate (CTA), nitrocellulose (NC), cellulose immobilized with dextran sulfate, and the like; and as the synthetic resin, there may be preferably used, polyacrylonitrile (PAN), polyimide (PI), polyester-based polymer alloy (PEPA), polystyrene (PS), polysulfone (PSF), polyethylene terephthalate (PET), polymethyl methacrylate (PMMA), polyvinyl alcohol (PVA), polyurethane (PU), ethylene vinyl alcohol (EVAL), polyethylene (PE), polyester, polypropylene (PP), polyvinylidene fluoride (PVDF), polyether sulfone (PES), polycarbonate (PC), cycloolefin polymer (COP), polyvinyl chloride (PVC), polytetrafluoroethylene (PTFE), ultra-high molecular weight polyethylene (UHPE);, Polydimethylsiloxane (PDMS), acrylonitrile-butadiene-styrene resin (ABS) or Teflon ^{®}.

Examples of the antioxidant contained in the resin molding include an antioxidant of phenolic series having a radical scavenging action and an antioxidant of sulfur series · phosphorus series having a peroxide decomposing action.

The antioxidant of phenolic series captures peroxyl radicals generated during thermal oxidation as a radical scavenger. The antioxidant of phenolic series is excellent in thermal oxidation preventing action and is added to almost of plastics. The antioxidant of sulfur series · phosphorus series converts hydroperoxide (ROOH), a degradation product, into stable ROH.

Specifically, Sumilizer ^{®} (manufactured by Sumitomo Chemical Co., Ltd.) and Adecastab ^{®} (manufactured by ADEKA Co., Ltd.), and the like are mentioned.

The resin molding of the present application is, but is not particularly limited to, an apparatus and the like that is required to be provided in a biologically sterilized state, and more desirably, an apparatus that is required to be transparent, for example a cell culture vessel such as microwell plate, microplate, culture flask, a variety of biodevices such as microtube, chip, syringe, prefilled syringe or vial and the like, and preferably a cell culture vessel. The shape of the resin molding of the present application is, not particularly limited to, a flat plate shape, a curved surface shape, an uneven shape, and the like. In the case of cell culture vessel, it is usually shaped with a plurality of wells (recesses).

For example, in the case of cell culture vessel, there may be provided with a coating film having the ability to inhibit adhesion of biological materials, for example as described in WO 2014/196650 and WO 2016/093293, and a primer for cell culture as described in WO 2020/040247 as primer for cell culture at least on a portion of surface of the resin molding.

As the other coating film having the ability to inhibit adhesion of cells, a copolymerization of an ethylenically unsaturated monomer and a polysaccharide or its derivative may be used. Examples of ethylenically unsaturated monomers can include (meth) acrylic acid and its ester; vinyl acetate; vinylpyrrolidone; ethylene; vinyl alcohol; as well as one or more ethylenically unsaturated monomers selected from the group consisting of hydrophilic functional derivatives thereof. Examples of polysaccharide or derivatives thereof can include polymers of cellulosic series such as hydroxyalkyl cellulose (e.g., hydroxyethyl cellulose or hydroxypropyl cellulose), starch, dextran and curdlan.

The term "hydrophilic functional derivative" refers to an ethylenically unsaturated monomer having a hydrophilic functional group or structure. Examples of hydrophilic functional group or structure include betaine structure; amide structure; alkylene glycol residue; amino group; as well as sulfinyl group and the like.

The betaine structure means a monovalent or divalent group of a compound having an amphoteric center of a quaternary ammonium type cationic structure and an acidic anionic structure, for example includes a phosphorylcholine group: 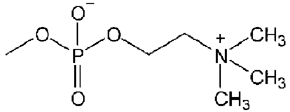 Examples of ethylenically unsaturated monomers having such structures can include 2-methacryloyloxyethyl phosphorylcholine (MPC) and the like.

The amide structure means the group represented by the following formula: 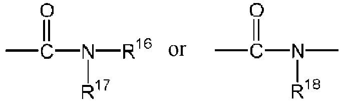 [wherein, R¹⁶, R¹⁷ and R¹⁸ are, independently to each other, a hydrogen atom or an organic group (for example, a methyl group, a hydroxymethyl group or a hydroxyethyl group, and the like)]. Examples of ethylenically unsaturated monomers having such structures can include (meth)acrylamide, N-(hydroxymethyl)(meth)acrylamide, and the like. Furthermore, monomers or polymers having such structures are disclosed, for example, in Japanese Patent Application Laid-Open No. 2010-169604.

The alkylene glycol residue means an alkyleneoxy group, (-Alk-O-) that remains after the hydroxyl groups at one or both ends of the alkylene glycol (HO-Alk-OH; wherein Alk is a C₁₋₁₀ alkylene group) undergo condensation reactions with other compounds, and also includes a poly (alkyleneoxy) group in which the alkyleneoxy unit is repeated. Examples of ethylenically unsaturated monomers having such structures can include 2-hydroxyethyl (meth)acrylate, methoxy polyethylene glycol (meth)acrylate, and the like. Furthermore, monomers or polymers having such structures are disclosed, for example, in Japanese Patent Application Laid-Open No. 2008-533489.

The amino group means a group represented by the formula: -NH₂, -NHR¹⁹ or - NR²⁰R²¹ [wherein, R¹⁹, R²⁰ and R²¹ are, independently to each other, organic groups (for example, a C₁₋₅ linear or branched alkyl group, and the like)]. The amino group in the present invention includes quaternized or chlorinated amino group. Examples of ethylenically unsaturated monomers having such structures can include dimethylaminoethyl (meth) acrylate, 2- (t-butylamino) ethyl (meth) acrylate, methacryloylcholine chloride, and the like.

The sulfinyl group means a group represented by the following formula: 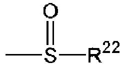 [wherein R²² is an organic group (for example, a C₁₋₁₀ organic group, preferably a C₁₋₁₀ alkyl group having 1 or more hydroxy groups, and the like)]. The polymer having such structures can include a copolymer disclosed in Japanese Patent Application Laid-Open No. 2014-48278 and the like.

The coating film having the ability to inhibit adhesion of the cells is normally formed with a film thickness (for example, 1 to 100 nm) that is thinner than the wavelength of visible light, and therefore is colorless and transparent to visible light.

### <Primary packaging (oxygen permeability) >

The material of the packaging material having oxygen permeability described above is, not particularly limited as long as the material has oxygen permeability described above, but is preferably the material having oxygen permeability while maintaining sterilizability, and is more preferably nonwoven fabric made of high density polyethylene. The term "nonwoven fabric" refers to as sheet-like fabric in which fibers are intertwined without weaving (In JIS L 0222, paper, felt or knitted fabric are not included.). The packaging material may be a single nonwoven fabric made of the high density polyethylene, or a combination with other materials (laminate film). The packaging material having oxygen permeability of the present application also includes a material in which a portion thereof is permeable to oxygen, for example, a material in which an opened portion of an oxygen impermeable laminate film is tape-sealed with a tape having oxygen permeability. In this case, the laminate film described above may be either oxygen permeable or impermeable. Therefore, the packaging material of the primary packaging may be a combination consisting of two or more packaging materials having oxygen permeability (laminate film) as well as a combination of a packaging material having oxygen permeability and an oxygen impermeable packaging material (for example, a laminate film of nonwoven fabric made of high density polyethylene and an oxygen impermeable packaging material described later, and a laminate film consisting of PET (polyethylene terephthalate)/LLDPE (Linear Low Density Polyethylene)).

The nonwoven fabric made of high density polyethylene may be used of a commercial product, and Tyvek ^{®} (manufactured by DuPont) is exemplified as a specific example. Tyvek ^{®} exists commercially in film form as well as tape. In the former JIS K 6748: 1995, high density polyethylene is polyethylene with density of 0.942 or more. In order to use as a packaging material, the packaging material of the present application is usually in the form of a film having a film thickness of about 150 ~ 200 µm per single material film. After the resin molding described above is packaged with the packaging material described above, the resin molding is sealed by heat sealing and the like.

### <Secondary packaging (oxygen impermeability) >

The oxygen impermeable packaging material described above is preferably a film comprising a material selected from the group consisting of nylon, polyvinyl alcohol, low density polyethylene, polypropylene, polystyrene, polyvinyl chloride, polyacrylic acid, polyacrylic ester, polyacrylonitrile, polyvinyl acetate, polyacrylamide, polydimethylsiloxane, and polyethylene terephthalate, among which a film containing nylon and/or low density polyethylene is more preferable. These may be films made of a single material, but it is preferable to use them as a laminate film made of two or more materials for the purpose of heat seal ability and physical resistance, and the like. In the former JIS K 6748: 1995, low density polyethylene is polyethylene having a density of 0.910 or more and less than 0.930. In order to use as the packaging material, the packaging material of the present application is usually in the form of a film having a film thickness of about 10 ~ 200 µm per single material film.

The primarily packaged resin molding described above is secondarily packaged with the packaging material described above, farther, the deoxidizer described below is placed thereinto, and then the resin molding is sealed by heat sealing and the like.

Regarding the oxygen permeability/oxygen impermeability, it is as described above.

### <Deoxidizer>

The deoxidizer in the present application is not particularly limited as long as the deoxidizing agent has oxygen absorbability. A commercially available product may be used, and a specific example thereof includes Ageless ^{®} (manufactured by Mitsubishi Gas Chemical Company, Inc.).

### <Radiation irradiation>

The radiation treatment of the present application is usually performed at an ambient temperature (for example, from about 0°C to about 40°C, preferably from about 10°C to about 30°C, more preferably about 25°C) after the secondary packaging, as is, or after tertiary packaging in which the secondary packaging is then further boxed with a corrugated cardboard and the like. The radiation treatment is preferably conducted by γ-rays, X-rays or electron beams irradiation as long as the sterilization can be completed. The radiation treatment is preferably by irradiation of γ-rays or X-rays, and more preferably γ-rays. The irradiation dose of γ rays may be, for example, the dose employed in the ordinary sterilization process, for example, irradiation of about 5 to 40 kGy is sufficient, 10 to 25 kGy is preferable.

### <The method of producing the radiation-irradiated (sterilized) resin molding>

The method of producing the radiation-irradiated resin molding of the present application comprises a step of primarily packaging the resin molding before the radiation irradiation with the packaging material having oxygen permeability, and a step of subsequently secondarily packaging with the oxygen impermeable packaging material described above together with the deoxidizer, and then conducting the radiation irradiation.

Furthermore, regarding the resin molding, the oxygen permeability/impermeability, the primary packaging, the secondary packaging, the deoxidizer, and the radiation irradiation in the method of producing of the present application are the same as those described above, including their suitable aspects and the like.

The radiation irradiation is preferably conducted under anaerobic conditions. Specifically, as described above, the radiation irradiation is conducted after the inside of the primary packaging has become anaerobic conditions, for example, after the residual oxygen concentration in the primary packaging has become less than 0.5%.

It is preferable to comprise a step of opening the secondary packaging under the atmosphere, following the radiation irradiation step described above. The time of opening under the atmosphere described above is, for example, 1 hour or more, 2 hours or more, 3 hours or more, 6 hours or more, 12 hours or more, and 24 hours or more.

By undergoing the step of producing described above, when the resin molding is transparent, a discoloration (yellowing) reduced, radiation-irradiated (sterilized) packaged resin molding can be produced.

The resin molding described above obtained by the method of producing of the present application is, but is not particularly limited to, an apparatus and the like that is required to be provided in a biologically sterilized state, and more desirably, is an apparatus that is required to be transparent, cell culture vessels such as microwell plates, microplates, culture flasks, a variety of biodevices such as microtubes, chips, syringes, prefilled syringes or vials, and preferably cell culture vessels.

### <Method of sterilizing resin molding>

The sterilization method of the resin composition of the present application comprises the step of primarily packaging the resin molding with the packaging material having oxygen permeability, a step of secondarily packaging with an oxygen impermeable packaging material together with the deoxidizer, and a step of conducting the radiation irradiation.

Furthermore, regarding the resin molding, the oxygen permeability/impermeability, the primary packaging, the secondary packaging, the deoxidizer, and the radiation irradiation in the method of the present application are the same as those described above, including their suitable aspects.

The radiation irradiation is preferably conducted under anaerobic conditions. Specifically, as described above, the radiation irradiation is conducted after the inside of the primary packaging has become anaerobic, for example, after the residual oxygen concentration in the primary packaging has become less than 0.5%

The method of the present application preferably comprises the step of opening the secondary packaging under the atmosphere, following the radiation irradiation step described above. The time of opening under the atmosphere is, for example, 1 hour or more, 2 hours or more, 3 hours or more, 6 hours or more, 12 hours or more, or 24 hours or more.

According to the method described above, when the resin molding is transparent, the discoloration (yellowing) due to the radiation irradiation is reduced.

### Examples

Hereinafter, the present invention will be illustrated in more detail based on examples, test examples and the like, but the present invention is not limited thereto.

### <Preparation Example 1>

### (Preparation of low cell adhesion coating material)

5.00 g of acid phosphoxyethyl methacrylate (Product name: Phosmer M, manufactured by UNICHEMICAL Co., Ltd., 91.8% of nonvolatile at 100°C for 1 hour by drying method, a mixture of acid phosphoxyethyl methacrylate (44.2 mass%), bis [2-(methacryloyloxy)ethyl] phosphate (28.6 mass%), and other substances (27.2 mass%)) was added to 7.97 g of ethanol and 23.92 g of pure water and dissolved by stirring, and 3.82 g of 2- (dimethylamino) ethyl methacrylate (manufactured by Tokyo Chemical Industry Co., Ltd.) and 0.04 g of 2,2'-azobis(N-(2-carboxyethyl)-2-methylpropionamidine) n-hydrate (Product name: VA-057, manufactured by Fujifilm Wako Pure Chemical Industries, Ltd.) were added sequentially while keeping the temperature at 20°C or below. The mixture containing all of the above ingredients, which was sufficiently stirred to become homogeneous, was introduced into a dropping funnel. On the other hand, 47.84 g of pure water was separately put into a three-necked flask with a cooling tube, which was nitrogen flowed and heated to the reflux temperature with stirring. While maintaining this state, the dropping funnel into which the mixture was introduced was set in the three-necked flask, and the mixture was added dropwise into the boiling liquid over one hour. After the completion of the dropwise addition, the resultant mixture was heated and stirred for 24 hours while maintaining the above environment, 88.60 g of a copolymer-containing varnish having a solid content of about 9.70 mass% was obtained. To 5.00 g of the copolymer-containing varnish obtained as described above, 56.39 g of pure water, 28.50 g of ethanol, and 0.85 g of a 1 mol/L aqueous solution of sodium hydroxide (1N) (manufactured by Kanto Kagaku Co., Ltd.) were added and sufficiently stirred to prepare a low cell adhesion coating material.

### <Preparation Example 2>

### (Preparation of a cell culture plate with low cell adhesion coating)

The low cell adhesion coating material obtained in Preparation Example 1 was added to separate wells of a 96 well cell culture plate 351172 made of polystyrene (manufactured by CORNING) so as to be 200 µL/well, and after allowed to stand still at room temperature for 1 hour, the excess varnish was removed. And it was dried in an oven at 50°C for 24 hours. Each coated well was then washed 3 times with 250 µL of pure water and dried using an oven at 50°C for 1 hour, thereby a cell culture plate with low cell adhesion coating was prepared.

### <Example 1>

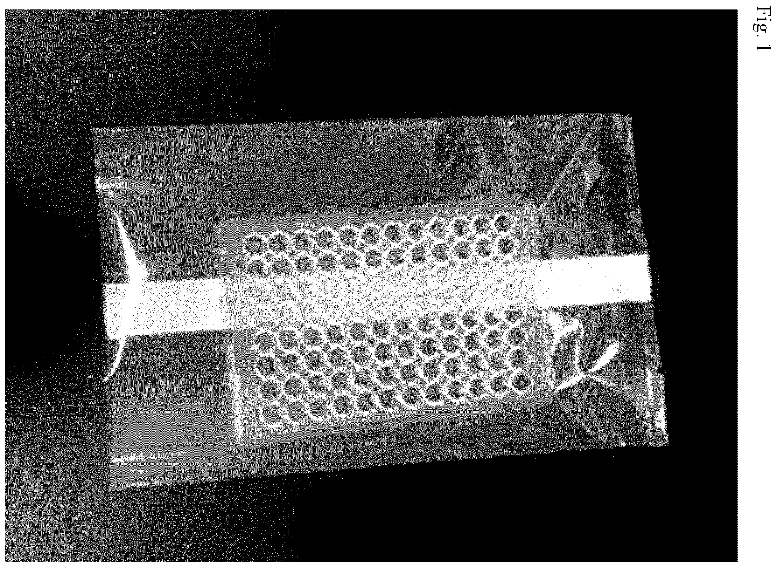

The lid of the outer container of the 24 well cell culture plate 142475 (manufactured by Thermo Fisher Scientific) (the material of the plate and the outer container; made of polystyrene) was opened in a sterile space, and the oxygen detector (Product name: Ageless Eye ^{®}, manufactured by Mitsubishi Gas Chemical Company, Inc.) was placed therein, and the lid was closed again. The plate was placed into a bag (135 × 200 mm) made from a laminate film comprised of PET (polyethylene terephthalate) (film thickness: 15 µm)/LLDPE (Linear Low Density Polyethylene) (film thickness: 50 µm) prepared by heat sealing, and its opening portion was tape-sealed with 20 mm width tape of Tyvek ^{®} (product number: 1073 B) (manufactured by DuPont) having oxygen permeability, and thereby the primary packaging was conducted (see FIG. 1).

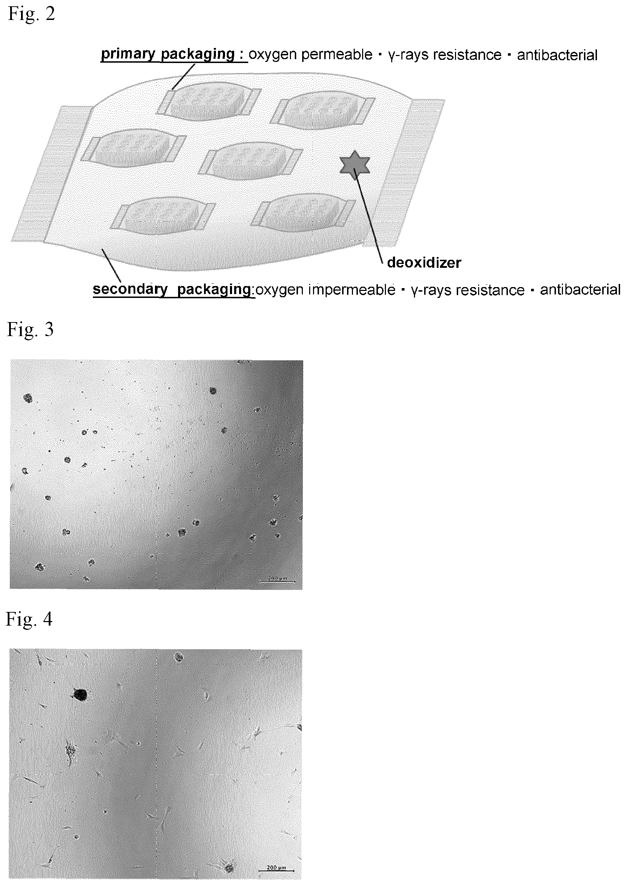

Then, this primarily packaged plate and the deoxidizer (Product name: Ageless ^{®}, manufactured by Mitsubishi Gas Chemical Company, Inc.) were placed into a bag (300 × 480 mm) made from a laminate film comprised of oxygen impermeable NY (nylon) (film thickness: 15 µm)/LLDPE (Linear low Density Polyethylene) (film thickness: 50 µm), and its opening portion was heat-sealed, and thereby the secondary packaging was conducted (see FIG. 2).

### <Example 2>

The lid of the outer container of the 6 well cell culture plate 351146 (manufactured by Corning Incorporated) (the material of the plate and the outer container; made of polystyrene) was opened in a sterile space, and the oxygen detector (Product name: Ageless Eye ^{®}, manufactured by Mitsubishi Gas Chemical Company, Inc.) was placed therein, and the lid was closed again. The plate was placed in a bag (135 × 200 mm) made from a laminate film comprised of NY (nylon) (film thickness: 15 µm)/LLDPE (Linear low Density Polyethylene) (film thickness: 50 µm) prepared by heat sealing, and its opening portion was tape-sealed with 20 mm width tape of Tyvek ^{®} (product number: 1073 B) (manufactured by DuPont) having oxygen permeability, and thereby the primary packaging was conducted (see FIG. 1).

Then, this primarily packaged plate and the deoxidizer (Product name: Ageless ^{®}, manufactured by Mitsubishi Gas Chemical Company, Inc.) were placed into a bag (300 × 480 mm) made from a laminate film comprised of oxygen impermeable NY (nylon) (film thickness: 15 µm)/LLDPE (Linear low Density Polyethylene) (film thickness: 70 µm), and its opening portion was heat-sealed, and thereby the secondary packaging was conducted (see FIG. 2).

### <Example 3>

The cell culture plate with low cell adhesion coating obtained in Preparation Example 2 was packaged under the same conditions as in Example 2.

### <Example 4>

The lid of the outer container of the 6 well cell culture plate 351146 (manufactured by Corning Incorporated) (the material of the plate and the outer container; made of polystyrene) was opened in a sterile space, and the oxygen detector (Product name: Ageless Eye ^{®}, manufactured by Mitsubishi Gas Chemical Company, Inc.) was placed therein, and the lid was closed again. The plate was primarily packaged with a bag (135 × 200 mm) laminated with Tyvek ^{®} having oxygen permeability.

Then, this primarily packaged plate and the deoxidizer (Product name: Ageless ^{®}, manufactured by Mitsubishi Gas Chemical Company, Inc.) were placed into a bag (300 × 480 mm) made from a laminate film comprised of oxygen impermeable NY (nylon) (film thickness: 15 µm)/LLDPE (Linear low Density Polyethylene) (film thickness: 70 µm), and its opening portion was heat-sealed, and thereby the secondary packaging was conducted (see FIG. 2).

### <Example 5>

The packaging was conducted under the same conditions as in Example 2, except that Falcon ^{®} Cell Culture 6 well multiwell plate 353046 (manufactured by CORNING) made of polystyrene for cell culture was used.

### <Example 6>

The packaging was conducted under the same conditions as in Example 2, except that a PrimeSurface ^{®} Dish 35 with low protein absorption treatment finished (manufactured by Sumitomo Bakelite Co., Ltd.) made of polystyrene was used.

### <Comparative Example 1>

The lid of the 24 well cell culture plate 142475 (manufactured by Thermo Fisher Scientific) (the material of the plate and the outer container; made of polystyrene) was opened in a sterile space, the oxygen detector (Product name: Ageless Eye ^{®}, manufactured by Mitsubishi Gas Chemical Company, Inc.) was placed therein, and the lid was closed again. The plate and the deoxidizer (Product name: Ageless ^{®}, manufactured by Mitsubishi Gas Chemical Company, Inc.) were placed into a bag (300 × 480 mm) made from a laminate film comprised of oxygen impermeable NY (nylon) (film thickness: 15 µm)/LLDPE (Linear Low Density Polyethylene) (film thickness: 50 µm), and its open portion was heat-sealed, and thereby packaging was conducted (corresponding only to the secondary packaging (oxygen impermeable) of Example 1).

### <Comparison Example 2>

The lid of the 24 well cell culture plate 142475 (manufactured by Thermo Fisher Scientific) was opened in a sterile space, and the oxygen detector (Product name: Ageless Eye ^{®}, manufactured by Mitsubishi Gas Chemical Company, Inc.) was placed therein, and the lid was closed again. The plate was placed into a bag (135 × 200 mm) made from a laminate film comprised of oxygen impermeable NY (nylon) (film thickness: 15 µm)/LLDPE (Linear low Density Polyethylene) (film thickness: 50 µm), and its opening portion was heat-sealed, and thereby the primary packaging was conducted.

Then, this primarily packaged plate and the deoxidizer (Product name: Ageless ^{®}, manufactured by Mitsubishi Gas Chemical Company, Inc.) were placed into a bag (300 × 480 mm) made from a laminate film comprised of oxygen impermeable NY (nylon) (film thickness: 15 µm)/LLDPE (Linear low Density Polyethylene) (film thickness: 50 µm), and its opening portion was heat-sealed, and thereby the secondary packaging was conducted (the primary packaging and the secondary packaging were conducted by using oxygen impermeable packaging materials).

### <Comparison Example 3>

The lid of the 24 well cell culture plate 142475 (manufactured by Thermo Fisher Scientific) was opened in a sterile space, and the oxygen detector (Product name: Ageless Eye ^{®}, manufactured by Mitsubishi Gas Chemical Company, Inc.) was placed therein, and the lid was closed again. The plate was placed into a bag (135 × 200 mm) made from a laminate film comprised of PET (polyethylene terephthalate) (film thickness: 15 µm)/LLDPE (Linear low Density Polyethylene) (film thickness: 50 µm) prepared by heat sealing, and its opening portion was tape-sealed with a 20 mm width tape of Tyvek^{®} (product number: 1073 B) (manufactured by DuPont) having oxygen permeability, and thereby the primary packaging was conducted (see FIG. 1) (corresponding only to the primary packaging of Example 1 (oxygen permeable)).

### <Comparison Example 4>

The lid of the outer container of the 6 well cell culture plate 351146 (manufactured by Corning Incorporated) (the material of the plate and the outer container; made of polystyrene) was opened in a sterile space, and the oxygen detector (Product name: Ageless Eye ^{®}, manufactured by Mitsubishi Gas Chemical Company, Inc.) and the deoxidizer (Product name: Ageless^{®}, manufactured by Mitsubishi Gas Chemical Company, Inc.) was placed therein, and the lid was closed again. The plate was placed into a bag (300 × 480 mm) made from a laminate film comprised of oxygen impermeable NY (nylon) (film thickness: 15 µm)/LLDPE (Linear low Density Polyethylene) (film thickness: 70 µm), and its opening portion was heat-sealed, and thereby only the primary packaging was conducted.

### <Comparative Example 5>

The lid of the outer container of the 6 well cell culture plate 351146 (manufactured by Corning Incorporated) (the material of the plate and the outer container; made of polystyrene) was opened in a sterile space, and the oxygen detector (Product name: Ageless Eye ^{®}, manufactured by Mitsubishi Gas Chemical Company, Inc.) was placed therein, and the lid was closed again. The plate was placed into a bag (135 × 200 mm) made from a laminate film comprised of oxygen impermeable NY (nylon) (film thickness: 15 µm)/LLDPE (Linear low Density Polyethylene) (film thickness: 70 µm), and its opening portion was heat-sealed, and thereby the primary packaging was conducted (see FIG. 1).

Then, this primarily packaged plate and the deoxidizer (Product name: Ageless ^{®}, manufactured by Mitsubishi Gas Chemical Company, Inc.) were placed into a bag (300 × 480 mm) made from a laminate film comprised of oxygen impermeable NY (nylon) (film thickness: 15 µm)/LLDPE (Linear low Density Polyethylene) (film thickness: 70 µm), and its opening portion was heat-sealed, and thereby the secondary packaging was conducted (see FIG. 2).

### <Comparative Example 6>

The primary and secondary packaging were conducted in the same manner as in Example 2.

### <Comparative Example 7>

The lid of the outer container of the cell culture plate with low cell adhesion coating obtained in Preparation Example 2 therein (material of the plate and the outer container; made of polystyrene) was opened in a sterile space, and the oxygen detector (Product name: Ageless Eye ^{®}, manufactured by Mitsubishi Gas Chemical Company, Inc.) and the deoxidizer (Product name: Ageless ^{®}, manufactured by Mitsubishi Gas Chemical Company, Inc.) were placed therein, then the lid was closed again. The plate was placed into a bag (135 × 200 mm) made from a laminate film comprised of NY (nylon) (film thickness: 15 µm)/(linear low density polyethylene) (film thickness: 50 µm) prepared by heat sealing, and its open portion was tape-sealed with 20 mm width tape of Tyvek ^{®} (product number: 1073 B) (manufactured by DuPont) having oxygen permeability, and thereby only the primary packaging was conducted.

### <Comparative Example 8>

The primary packaging was conducted under the same conditions as in Comparative Example 4, except that Falcon ^{®} Cell Culture 6 well multi-well plate 353046 (manufactured by CORNING) made of polystyrene for cell culture was used in a sterile space.

### <Comparative Example 9>

The primary packaging was conducted under the same conditions as in Comparative Example 4, except that PrimeSurface ^{®} Dish 35 (manufactured by Sumitomo Bakelite Co., Ltd.) with low protein absorption treatment finished made of polystyrene was used in a sterile space.

Table 1 shows the constituent of containers and packaging in Examples and Comparative Examples, and the presence or absence of radiation irradiation.

**[Table 1]**

| | Container | Primary packaging | Secondary packaging | γ-rays irradiation 25 kGy |
|---|---|---|---|---|
| Example 1 | Non-treated Plate 142475(24 well) | Oxygen permeable | Oxygen impermeable + Ageless | Presence |
| Example 2 | Non-treated Plate 351146(6 well) | Oxygen permeable | Oxygen impermeable + Ageless | Presence |
| Example 3 | Preparation Example 2 | Oxygen permeable | Oxygen impermeable + Ageless | Presence |
| Example 4 | Non-treated Plate 351146(6 well) | Oxygen permeable only Tyvek | Oxygen impermeable + Ageless | Presence |
| Example 5 | TC Plate 353046(6 well) | Oxygen permeable | Oxygen impermeable + Ageless | Presence |
| Example 6 | PrimeSurface Dish 35 | Oxygen permeable | Oxygen impermeable + Ageless | Presence |
| Comparative Example 1 | Non-treated Plate 142475(24 well) | - | Oxygen impermeable + Ageless | Presence |
| Comparative Example 2 | Non-treated Plate 142475(24 well) | Oxygen impermeable | Oxygen impermeable + Ageless | Presence |
| Comparative Example 3 | Non-treated Plate 142475(24 well) | Oxygen permeable | - | Presence |
| Comparative Example 4 | Non-treated Plate 351146(6 well) | Oxygen impermeable + Ageless | - | Presence |
| Comparative Example 5 | Non-treated Plate 351146(6 well) | Oxygen impermeable | Oxygen impermeable + Ageless | Presence |
| Comparative Example 6 | Non-treated Plate 351146(6 well) | Oxygen permeable | Oxygen impermeable + Ageless | Absence |
| Comparative Example 7 | Preparation Example 2 | Oxygen permeable + Ageless | - | Presence |
| Comparative Example 8 | TC Plate 353046(6 well) | Oxygen impermeable + Ageless | - | Presence |
| Comparative Example 9 | PrimeSurface Dish 35 | Oxygen impermeable + Ageless | - | Presence |

### (γ-ray irradiation treatment)

The well plate packaged under the conditions described above was packaged with a corrugated cardboard, and γ-ray irradiation treatment was conducted at 25 kGy under ambient temperature and atmosphere. Still regarding only Comparative Example 6, γ-ray irradiation was not conducted.

### (Confirmation of residual oxygen concentration using the oxygen detector)

After γ-ray irradiation, the color of the oxygen detector (Product name: Ageless Eye ^{®}, manufactured by Mitsubishi Gas Chemical Company, Inc.) placed inside of the lid of the well plate described in Example was confirmed (without opening the packaging). Since the color changes to pink when the oxygen concentration is less than 0.1% and to blue when the oxygen concentration is 0.5% or more, it is confirmed that the oxygen concentration is less than 0.5% by being close to pink instead of blue. The results are shown in Table 2.

### (Direct measurement of residual oxygen concentration after γ-ray)

Residual oxygen concentration was measured by extracting gas from the inside of the primary package without opening the secondary packaging and the primary packaging of the well plate after γ-ray irradiation, using a residual oxygen concentration meter (Product name: Packmaster RO -103, manufactured by Iijima Electronics corporation). The results are shown in Table 3.

### (Confirmation of the color of the well plate)

The color of the well plate was visually confirmed without opening the secondary packaging and the primary packaging after γ-ray irradiation. Furthermore, in Example 1 and Comparative Example 2 having the secondary packaging, the color was similarly confirmed visually after the packaging was removed and left to stand in the atmosphere at room temperature for 24 hours. The results are shown in Table 2.

In addition, discoloration of the well plate was confirmed by visually observing and from an amount of increase of the value of b * obtained by a spectrophotometer (Name: CM -3700 A, manufactured by KONICA MINOLTA, INC.). Examples and Comparative Examples having secondary packaging were confirmed after the secondary packaging was removed and left to stand in ambient air at room temperature for 24 hours. The results are shown in Table 3. It can be confirmed that the higher the value of b *, the more yellowing.

### (Confirmation of Bioburden)

Although Example 2 and Comparative Example 6 were packaged under the same conditions, there was a difference between them with and without γ-ray irradiation. To each of the interior of these two plates, 1 mL of a biological indicator (10⁶ of bacteria count) (Product name: Spore Suspension for γ-ray sterilization B. pumilus (27142), bacteria count 10⁶/mL, manufactured by AR BROWN LIFE SCIENCE) was intentionally added, and only Example 2 was subjected to γ-ray sterilization at 25 kGy. The two types of plates were then subjected to a bacterial count test by the collecting method. The results are shown in Table 3. Furthermore, since the test with the collecting method could not count 100 cfu/g or more, regarding Comparative Example 6, 10⁵ times dilution is conducted to evaluate. That is, cfu multiplied by 10⁵ times of the actual bacteria count is described in Table 2. Example 2 is evaluated without dilution.

### (Cell adhesion test)

The cells were used of mouse embryonic fibroblasts C3H10T1/2 (manufactured by DS Pharma Biomedical). The medium used for cell culture was used of BME medium (Thermo Fisher Scientific) containing 10% FBS (manufactured by Sigma-Aldrich) and L-glutamine-penicillin-streptomycin stabilized solution (manufactured by Thermo Fisher Scientific). The cells were incubated in static culture in a 37°C/CO ₂ incubator for 2 days or more using petri dish (10 mL of medium) with a diameter of 10 cm in a state of maintaining a 5% carbon dioxide concentration. Subsequently, the cells were washed with 5 mL of PBS, then 1 mL of 0.25 w/v% trypsin -1 mmol/L EDTA solution (manufactured by Fujifilm Wako Pure Chemical Corporation) was added to peel off the cells thereby, and the cells were suspended in each of 10 mL of the medium described above. After centrifugation of the suspension (TOMY SEIKO Co., Ltd., Model No. LC - 200, 1000 rpm/3 minutes, at room temperature), the supernatant was removed, and the above medium was added to prepare a cell suspension. To each well of the plates obtained in Example 3 (the one in which the secondary package is opened to the atmosphere after γ-ray irradiation, and the primary package is opened at least 24 hours after) and the plate obtained in Comparative Example 7 (the one in which the primary package was opened at least 24 hours after γ-ray irradiation), 150 µL of the cell suspension was added so that the cell suspensions would be 1 × 10⁴ cells/well. Thereafter, the plates were left to stand in a CO ₂ incubator at 37°C for 3 days in the state of maintaining a 5% carbon dioxide concentration.

After 3 days of culture, the adhesion of cells to each well of the plates prepared as described above was compared based on observation by an inverted microscope (Manufactured by Nikon Corporation, ECLIPSE TS 100 F) (magnification: 10 x). The results are shown in Table 3.

**[Table 2]**

| | Residual oxygen (%) @ after γ-rays irradiation | Color of well plate @ after γ-rays irradiation | Color of well plate @ after secondary packaging opened |
|---|---|---|---|
| Example 1 | <0.5% | Yellowing | Transparent after 24 hour |
| Comparative Example 1 | <0.5% | Yellowing | Yellowing |
| Comparative Example 2 | <0.5% | Yellowing | Yellowing |
| Comparative Example 3 | ≧0.5% | Transparent | Transparent |

**[Table 3]**

| | After γ rays residual oxygen (%) | Before γ rays visual ovservation/ value of b* | After γ rays visual ovservation/ value of b* | After γ rays confirmed bacterial count [cfu] | After γ rays cell adhesion test |
|---|---|---|---|---|---|
| Example 2 | <0.1 | Transparent /0.4 | Transparent /0.5 | 0 | - |
| Example 3 | <0.1 | Transparent /0.4 | Transparent /0.5 | - | Low cell adhesion |
| Example 4 | <0.1 | Transparent /0.4 | Transparent /0.5 | - | - |
| Example 5 | <0.1 | Transparent /0.5 | Transparent /0.5 | - | - |
| Example 6 | <0.1 | Transparent /0.2 | Transparent /0.2 | - | - |
| Comparative Example 4 | <0.1 | Transparent /0.4 | Yellow/2.4 | - | - |
| Comparative Example 5 | 21.0 | Transparent /0.5 | Yellow/1.0 | - | - |
| Comparative Example 6 | 21.0 | Transparent /0.5 | Transparent /0.4 | 8×10⁵ | - |
| Comparative Example 7 | 21.0 | Transparent /0.4 | Transparent /0.5 | - | Cell adhesion |
| Comparative Example 8 | <0.1 | Transparent /0.5 | Yellow/2.1 | - | - |
| Comparative Example 9 | <0.1 | Transparent /0.2 | yellow/1.5 | - | - |

As shown in the results of Table 3, the plate packaged in Example 2 showed no yellowing also after γ-ray irradiation and maintained a bioburden (bacteria count) of 1 cfu/g or less. The plate packaged in Example 3 was able to suppress yellowing, and low cell adhesion was confirmed (see FIG. 3). The plate packaged in Example 4 could suppress yellowing even the primary packaging was with only oxygen permeable Tyvek. Further, in Examples 5 and 6, the same inhibition of yellowing could be achieved even in the cell culture plates different from those of Examples 1 to 4.

The plate packaged in Comparative Example 4 showed obvious yellowing. The plate packaged in Comparative Example 5 showed slight yellowing. The plate packaged in Comparative Example 6 did not show yellowing, but was not properly sterilized. The plate packaged in Comparative Example 7 (negative control of Example 3) showed no yellowing but low cell adhesion was inhibited (see FIG. 4). Regarding the plates packaged in Comparative Examples 8 and 9 (negative object of Examples 5 and 6), yellowing was confirmed.

From the above, it has been proved that the sterilization method of the resin molding of the present application is the method of a γ-ray sterilization capable of securing yellowing resistance and sterilizability of the resin molding such as the cell culture plate.

### Industrial applicability

According to the present invention, it is possible to produce a sterilized resin molding, for example, the sterilized cell culture vessel, without discoloration such as yellowing.

## Claims

1. A radiation-irradiated and packaged resin molding, comprising that said resin molding is primarily packaged with a packaging material having oxygen permeability, and then secondarily packaged with an oxygen impermeable packaging material together with a deoxidizer.

2. The resin molding according to claim 1, wherein the inside of the primary packaging is radiation-irradiated under anaerobic conditions.

3. The resin molding according to claim 1 or 2, wherein the inside of the primary packaging is radiation-irradiated under residual oxygen concentration of less than 0.5%.

4. The resin molding according to any one of claims 1 to 3, wherein said secondary packaging is opened under the atmosphere after the radiation irradiation.

5. The resin molding according to claim 4, wherein discoloration after said radiation irradiation is reduced.

6. The resin molding according to any one of claims 1 to 5, wherein the packaging material of said primary packaging comprises nonwoven fabric made of high density polyethylene.

7. The resin molding according to any one of claims 1 to 6, wherein the packaging material of said primary packaging comprises a combination of the packaging material having oxygen permeability and the oxygen impermeable packaging material.

8. The resin molding according to any one of claims 1 to 7, wherein the packaging material of said primary packaging comprises a combination of nonwoven fabric made of high density polyethylene having oxygen permeability and the oxygen impermeable packaging material.

9. The resin molding according to any one of claims 1 to 8, wherein said oxygen impermeable packaging material is selected from the group consisting of nylon, polyvinyl alcohol, low density polyethylene, polypropylene, polystyrene, polyvinyl chloride, polyacrylic acid, polyacrylate, polyacrylonitrile, polyvinyl acetate, polyacrylamide, polydimethylsiloxane, and polyethylene terephthalate.

10. The resin molding according to any one of claims 1 to 9, wherein said radiation is selected from γ-rays, X-rays or electron rays.

11. A method of producing a radiation-irradiated and packaged resin molding, comprising a step of primarily packaging a resin molding with a packaging material having oxygen permeability, and a step of subsequently secondarily packaging them with an oxygen impermeable packaging material together with a deoxidizer, thereafter conducting radiation irradiation.

12. The producing method according to claim 11, wherein the step of the radiation irradiation is conducted under anaerobic conditions.

13. The producing method according to claim 11 or 12, comprising a step of opening the secondary package under the atmosphere, following said step of the radiation irradiation.

14. The producing method according to claim 13, wherein discoloration after the radiation irradiation is reduced.

15. The resin composition according to any one of claims 1 to 10, wherein said resin molding is a cell culture vessel.

16. The producing method according to any one of claims 11 to 14, wherein said resin molding is a cell culture vessel.

17. The resin composition according to any one of claims 1 to 10, and 15, wherein a bioburden of said resin molding is 1 cfu/g or less.

18. The method of producing according to any of claims 11 to 14, and 16, wherein a bioburden of said resin molding after the step of the radiation irradiation is 1 cfu/g or less.

19. A method of sterilizing a resin molding, comprising:
a step of primarily packaging a resin molding with a packaging material having oxygen permeability;
a step of secondarily packaging with an oxygen impermeable packaging material together with a deoxidizer; and
a step of conducting radiation irradiation.

20. The method according to claim 19, wherein the radiation irradiation is conducted with the inside of the primary packaging being under anaerobic conditions.

21. The method according to claim 19 or 20, comprising a step of opening the secondary package under the atmosphere, following the step of the radiation irradiation.

22. The method according to claim 21, wherein discoloration of the resin composition after radiation irradiation is reduced.
